# EUROPEAN PATENT APPLICATION

(11) **EP 3 124 090 A1**
(43) Date of publication of application: **01.02.2017**
(21) Application number: 16169037.5
(22) Date of filing: 08.03.2006
(51) Int. Cl.: A63D 15/00, A61K 31/522, A61K 31/401

(54) **DIETS FOR REDUCING LEVELS OF DISEASE ASSOCIATED PROTEINS**

(30) Priority: 03.05.2005 US 123706
(62) Divisional of application: 06737807.5
(71) Applicant: Accera, Inc., Broomfield, Colorado 80021 (US)
(72) Inventor: HENDERSON, Samuel T., Boulder, Colorado 80301 (US)
(74) Representative: Salisbury, Frances

(57) **Abstract**

The subject invention concerns the reduction of protein aggregation in the neurons of a mammal through the use of a ketogenic treatment such as a ketogenic diet, a physical training regimen and/or administration of agents to increase fatty acid oxidation. Such ketogenic treatment can be useful in the reduction of certain aggregates including amyloid β peptide, polyglutamine containing huntintin protein, polyglutamine containing androgen receptor, polyglutamine containing atrophin-1, polyglutamine containing ataxins, a-synclein, prion protein, tau and superoxide dismutase 1 (SOD1).

## Description

### FIELD OF THE INVENTION

The present invention relates to the reduction of accumulated proteins in a mammal. In particular, the present invention relates to the reduction of amyloid beta peptides in a mammal, and is applicable to other diseases associated with accumulation of proteins, such as Huntington's disease, Parkinson's disease, Prion diseases, taupathologies, amytrophic lateral sclerosis and others.

### BACKGROUND OF THE INVENTION

There are a number of diseases associated with accumulation of protein products. For example, Alzheimer's disease is associated with accumulation of the amyloid beta peptide. Huntington's disease is associated with accumulation of polyglutamine containing aggregates of the Huntingtin protein. Familial forms of Parkinson's disease are associated with aggregates of alpha-synuclein. Prion diseases are associated with aggregates of the Prion protein (PrP). Amytrophic lateral sclerosis is associated with accumulation of mutant superoxide dismutase (SOD1) protein. At this time, there are no effective treatments or preventative measures for these diseases. It is the novel insight of the inventor that, contrary to prevailing views, high fat / low carbohydrate (ketogenic) diets will be effective treatments for said diseases.

### Alzheimer's disease

Alzheimer's disease (AD) is a progressive, neurodegenerative disorder that primarily strikes the elderly. AD is very common, affecting as many as 47% of those between the ages of 75 to 84 years old (Evans, et al., Jama, 1989, 262:2551-6). The clinical course of AD typically begins in the seventh or eighth decade and is characterized by disturbances in memory, language, and spatial skills, all of which worsen as the disease progresses. AD is frequently accompanied by behavioral symptoms, such as anxiety and depression. Pathologically, AD is characterized by accumulation of senile plaques, dystrophic neurites, neurofibrillar tangles, as well as gross structural changes, such as loss of neurons in the hippocampus, nucleus basalis and other areas. There are no effective treatments and the disease invariably progresses until death.

Analysis of the senile plaques revealed that they contained large amounts of the amyloidic peptide Aβ, derived from cleavage of the amyloid precursor protein (APP). Subsequently, mutations in APP were found to be causative in cases of familial AD. In addition, mutations in APP and the presenilin genes (PS 1 and PS2) were found to result in increased generation of the more amyloidic form of Aβ (Aβ 42). The connection between pathology and genetics led to hypothesis that Aβ is central to the etiology of AD (for overview see Selkoe, J. Neruopathol. Exp. Neurol., 1994, 53:438-447). However, recent evidence demonstrates that APP functions as a vesicular transport protein and the etiology of the disease may not be related to the toxicity of Aβ peptides, but instead caused by failure of APP to efficiently move vesicles within the axon (Stokin, et al., Science, 2005, 307:1282-8). Such data brings much of the amyloid cascade hypothesis into doubt.

While the precise etiology of AD remains unclear, both the pathology and genetics suggest a clear role for APP and its processing machinery in AD. In particular, a large body of evidence demonstrates that accumulation of Aβ serves, at least, as a marker for the disease (see Selkoe, Ann Intern Med, 2004, 140:627-38). Thus, strategies to limit Aβ production or enhance Aβ clearance have been investigated as potential therapies. For example, in mouse models of AD, decreasing Aβ levels by immune mediated clearance improves cognitive performance, suggesting that decreasing Aβ may serve as an effective treatment for the disease (Schenk, et al., Nature, 1999, 400:173-7). Unfortunately, vaccinations with Aβ in humans caused CNS inflammation and the clinical trials had to be halted (Robinson, et al., Neurobiol Aging, 2004, 25:609-15). Therefore, treatments for AD are desperately needed.

### High fat diets linked to Alzheimer's disease

Several authors have attributed AD and the accumulation of Aβ to dietary factors. In particular, high fat diets have been repeatedly implicated as increasing risk for development of AD, while low fat diets have been proposed as protective (Grant, Alz. Dis. Rev., 1997, 42-55;Kalmijn, et al., Ann Neurol, 1997, 42:776-82). For example, Kalmijn *et al.* examined the eating habits of 5,386 residents of Rotterdam, Netherlands and state: "After adjustment for age, sex, education, and energy intake, high intakes of the following nutrients were associated with an increased risk of dementia: total fat (RR= 2.4 [1.1-5.2]), saturated fat (RR = 1.9 [0.9-4.0]), and cholesterol (RR = 1.7 [0.9-3.2]).....This study suggests that a high saturated fat and cholesterol intake increases the risk of dementia, whereas fish consumption may decrease this risk." (Kalmijn, et al., Ann Neurol, 1997, 42:776-82). Other demographic studies have seemed to confirm this link. For example, Morris *et al.* studied the eating habits of 815 subjects greater than 65 years of age and correlated macronutrient intake with subsequent development of dementia. These authors concluded that intake of saturated fats may increase the risk for dementia (Morris, et al., Arch Neurol, 2003, 60:194-200).

In addition, several mouse studies have seemed to confirm the link between lipid rich diets and AD (Ho, et al., Faseb J, 2004, 18:902-4;Refolo, et al., Neurobiol Dis, 2000, 7:321-31). Both Refolo *et al.* and George *et al.* tested high cholesterol diets, wherein high levels of cholesterol were added to the standard diet. Refolo *et al.* crossed transgenic mice (Tg2576), which express the familial AD mutant human APP (K670N, M671L) gene, with mice expressing the human familial mutant PS1 (M146V) gene, to generate double transgenic PSAPP animals. 7 PSAPP animals were fed a standard diet (Purina Test Diet No 5755C: 0.005% cholesterol, 10% fat, and 3.6 kcal/g), and 9 were fed a high cholesterol diet (Purina Test Diet No. 5801C: 5% cholesterol, 10% fat, 2% sodium cholate and 5.2 kcal/g). Aβ levels were measured both in sera and CNS. The researchers concluded: "Our data showed that diet-induced hypercholesterolemia resulted in significantly increased levels of formic acid-extractable Aβ peptides in the CNS. Furthermore, the levels of total Aβ were strongly correlated with the levels of both plasma and CNS total cholesterol." (Refolo, et al., Neurobiol Dis, 2000, 7:321-31).

Ho *et al.* studied a high fat diet in a similar mouse model of AD. Ho *et al.* raised female Tg2576 (APP K670N, M671L) mice on either a standard rodent diet (10% fat, 70% carbohydrate, 20% protein) or a high fat diet (60% fat, 20% carbohydrate, 20% protein) for 9 months, then examined the levels of Aβ in the brain. Ho *et al.* report that high fat diets increased Aβ load greater than 2 fold when compared to the standard diet (Ho, et al., Faseb J, 2004, 18:902-4).

### High Fat diets linked to decreased cognitive performance

Studies in rats have linked the consumption of high-fat diets to poor cognitive performance. In a series of studies rats were fed diets rich in both saturated and polyunsaturated fats and cognitive performance measured by several tests. When fat intake was greater than 20% by weight of the chow, rats showed significantly lower performance in a variety of tasks, including Olton's radial arm maze, a non-spatial test of conditional associative learning, and the variable-interval, delayed alternation (VIDA) test, a test of rule learning and memory function. Animals were fed either low fat chow (4.5% fat by weight), or a high fat chow (20% fat by weight). The high fat diets impaired all of these tests, and suggested a general decline in cognitive function (Winocur and Greenwood, Behav Brain Res, 1999, 101:153-61).

In addition, high fat diets have been implicated in poor recovery from brain injury (Wu, et al., Neuroscience, 2003, 119:365-75). Animals were fed either standard chow or high fat chow (39% of energy derived from fat, 40% from carbohydrate), after 4 weeks on the diet the animals were subjected to a fluid percussion injury (FPI) to the brain of 1.5 atm. The animals remained on the test diets for seven more days then cognitive testing was performed. As expected, FPI impaired the performance on Morris water maze test in both groups, but the impairment was much worse in the high fat fed group, leading to the suggestion that high fat diets are deleterious for recovery from brain injury.

As described above, both demographic and transgenic mouse studies have led to the widely held belief that high fat diets increase the risk of dementia, in particular AD. For example, in the Annual Review of Public Health, Haan and Wallace (2004), state "Vascular risk factors such as type 2 diabetes, hypertension, dietary fat intake, high cholesterol, and obesity have emerged as important influences on the risk of both vascular and Alzheimer's dementia." (Haan and Wallace, Annu Rev Public Health, 2004, 25:1-24).

### Parkinson's disease

Parkinson's disease (PD) is a common neurodegenerative disease that affects 1 in 100 people over the age of 60, with the average age of onset being 60 years of age in the United States. Clinically, PD is characterized by rest tremor of a limb (i.e. shaking with the limb at rest), slowness of movement (bradykinesia), rigidity (stiffness, increased resistance to passive movement) of the limbs or trunk, and poor balance (postural instability). Pathologically, PD is characterized by deposition of cytoplasmic inclusions called Lewy bodies within dopaminergic neurons. These inclusions are composed mainly of the protein alpha-synuclein, a product of the PARK1 gene. Similar to AD, accumulation of alpha-synuclein is thought to causative in at least some forms of the disease (Taylor, et al., Science, 2002, 296:1991-5). There are no effective treatments for PD.

As in the case of AD, most instances of PD cannot be traced to genetic mutations. Instead environmental influences have been implicated. For example, like AD, PD has also been linked to the consumption of a high fat diet. Johnson *et al.* examined the eating habits of 126 PD patients and 432 controls. The patients and controls were matched for age, education and body mass index and were required to complete a food questionnaire. PD patients were found to eat an average of 61.3 grams of fat versus 55 grams for the control group (p = 0.056). They concluded that high intake of total fat, saturated fats, cholesterol, lutein and iron increased the risk of developing PD (Johnson, et al., Int J Epidemiol, 1999, 28:1102-9).

### Polyglutamine diseases

There are several degenerative neurological disorders caused by expansion of polyglutamine encoding DNA regions. This class of diseases include Huntington's disease (HD; huntingtin), spinal and bulbar muscular atrophy (SBMA; androgen receptor), dentatorubral and pallidoluysian atrophy (DRPLA; atrophin-1), and a number of spinocerebellar ataxias (SCA; ataxins). In each of these disorders a genomic region of repeated CAG nucleotides becomes expanded, leading to the production of proteins containing long stretches of repeated glutamine amino acids (Q), referred to as polyglutamine or polyQ (Michalik and Van Broeckhoven, Hum Mol Genet, 2003, 12 Spec No 2:R173-86).

The polyglutamine containing proteins aggregate within the cells to form insoluble depositions of the mutant protein. Whether the deposits themselves are toxic or interference with the normal function of the affected protein is toxic remains an unanswered question. However, decreasing production or clearance of the deposits has been associated with improvement in the disease. Yamamoto *et al.* studied an inducible expression system in mice that could turn on and off a transgene expressing exon 1 of the Huntingtin gene containing a 94 polyQ repeat within the forebrain (Yamamoto, et al., Cell, 2000, 101:57-66). When the transgene was turned on, the mice developed progressive motor dysfunction, neuronal inclusions, and neuropathology typical of HD. If the transgene was subsequently turned off, the condition of the animals improved, i.e. motor dysfunction lessened and neuronal inclusions disappeared. The improvement in the health of the animals implies that continual supply of mutant protein is required for the progression of the disease. Furthermore, such experiments suggest reducing the production of mutant protein may alleviate the disease (Yamamoto, et al., Cell, 2000, 101:57-66). Since other polyQ associated diseases share deposits of mutant proteins, this implies a general strategy to combat such diseases.

### Prion diseases

There are several forms of neurodegenerative diseases that are classified as Prion diseases, including Creutzfeldt-Jacob disease, Gerstmami-Straussler-Scheinker disease, and fatal familial insomnia. Clinically, prion diseases show large variability in symptoms, including dementia, disordered movement, ataxia and insomnia. Pathologically, prion diseases are similar in that they feature accumulation of amyloid material containing the prion protein (PrP). Other pathological features include spongiform vacuolation, gliosis and neuronal loss. The accumulation of PrP is believed to be due to conversion of the normal form (PrP^{C}) into an abnormal form (PrP^{SC}). Interestingly, abnormal PrP^{SC} is thought to be capable of converting normal PrP^{C} protein into the abnormal aggregating PrP^{SC} form. The remarkable ability of PrP^{SC} to convert PrP^{C} into the mutant form is the proposed route of transmission of many prion diseases. Unlike other protein aggregation diseases, prions are believed to be orally transmissible across species, for example bovine spongiform encephalopathy (Mad Cow disease) is believed to infect humans by consumption of tainted meat (for overview see Taylor, et al., Science, 2002, 296:1991-5). There are no treatments for prion diseases.

### Taupathologies

Similar to Prion diseases a number of conditions are referred to as taupathologies. These include Pick's disease, corticobasal degeneration, progressive supranuclear palsy and amyotrophic lateral sclerosis/parkinsonism dementia complex. Each of these disorders is associated with filamentous tau containing inclusions within neurons. Such tau filaments are found in other degenerative disorders such as AD. A causative role for tau has been directly implicated in frontotemporal dementia, where mutations in the tau gene lead to aggregation of the tau protein and development of the disease (Taylor, et al., Science, 2002, 296:1991-5).

### Familial Amyotrophic Lateral Sclerosis

Amyotrophic Lateral Sclerosis (ALS) is a rapidly progressive, invariably fatal neurological disease that attacks motor neurons. Clinically, ALS causes muscle weakness with a wide range of progressively worsening symptoms. Due to the impairment of motor neuron function, ALS patients have difficulty with moving, swallowing (dysphagia), and speaking (dysarthria). Upper motor neuron degeneration is evident by tight and stiff muscles (spasticity) and exaggerated reflexes (hyperreflexia) including an overactive gag reflex. Lower motor neuron degeneration includes muscle weakness and atrophy, cramps, and twitches (fasciculations). Eventually, all voluntary muscles are affected, and patients lose the ability to move their arms, legs, and body. Muscles in the diaphragm and chest wall are also affected and patients eventually lose the ability to breathe without ventilatory support. Most people with ALS die from respiratory failure, usually within 3 to 5 years from the onset of symptoms.

In most cases of ALS the cause is unknown, however roughly 20% of familial cases are attributed to mutations in the SOD1 protein. In these cases, mutations in SOD1 result in aggregation of the protein. Similar to other protein aggregation diseases, the mutant protein is found in inclusions in neurons and glial cells. The cumulative evidence suggests that ALS is similar to other protein aggregate diseases in that it is the accumulation of large amounts of protein inclusions that are detrimental, and a general mechanism to clear such proteins is desperately needed.

### Ketogenic diets

Ketogenic diets were developed to mimic starvation without caloric deprivation and have been utilized in humans to treat epilepsy. The rationale for using a ketogenic diet to treat epilepsy is based on the long record of observations, dating back to the 5^{th} century BC, that fasting reduces seizures (for overview see Lefevre and Aronson, Pediatrics, 2000, 105:E46). During the 1920s ketogenic diets were developed that mimic starvation or fasting by maintaining subjects in extended periods of ketosis, measured as elevated serum ketone bodies (β-hydroxybutyrate, acetoacetate and acetone). These diets contained very low amounts of carbohydrates and protein and were devised to provide roughly 90% of calories from fats. The low carbohydrate and protein content greatly reduces insulin signaling and as a result induces high levels of fatty acid utilization and the production of ketone bodies, a hallmark of ketogenic diets.

Due to the high fat content of a ketogenic diet, it provides adequate calories, yet insulin signaling is low thereby mimicking a fasting condition. The low insulin signaling increases lipoprotein lipase activity in non-adipocyte tissues stimulating free fatty acid uptake and oxidation by tissues such as muscle and liver. Within the liver, the large amounts fatty acid oxidation leads to high levels of acetyl-CoA which is then used to synthesize ketone bodies. Since the liver lacks the enzymes to metabolize ketone bodies they are released into the bloodstream to be used by peripheral tissues. Thus, elevated ketone bodies in the blood are hallmark of conditions of decreased glucose availability and low insulin signaling.

Much like high fat diets, ketogenic diets have also been implicated as decreasing cognitive function in mammals. Zhao *et al.* raised rats on a low-carbohydrate, low-protein, ketogenic diet and tested them for several cognitive tasks. Those receiving the ketogenic chow did much worse. In these studies serum beta-hydroxybutyrate levels were significantly elevated in the ketogenic group (KD group 3.398 + 0.817 mmol/L vs regular chow 0.348 + 0.07 mM/L, p < 0.001). Animals receiving the ketogenic chow did dramatically worse in the Morris water maze test (F = 5.25, p = 0.003). In aggregate these studies led the authors to conclude "KD fed rats, with or without SE, had significantly impaired visual spatial learning and memory compared with rats that were fed regular diet." (Zhao, et al., Pediatr Res, 2004, 55:498-506).

Therefore, because of the association between high fat diets and dementia, ketogenic diets have not been investigated as treatment for protein aggregation diseases.

### SUMMARY OF THE INVENTION

The subject invention concerns a method for reducing protein aggregation in the brain of a mammal. This method comprises the administration of a ketogenic treatment so as to produce ketosis or a ketogenic state in the mammal for a predetermined period of time. Such treatment can substantially reduce the protein aggregation in the mammal's brain. In embodiments of increasing preference, the protein aggregation in the brain or neurons is decreased by at least 10%, 15%, 20%, 25%, 30% and increments increasing by 5% up to a 100% reduction as measured by weight over pre-treatment values. Ketosis or the ketogenic state is achieved when the serum ketone body level remains elevated above normal fasting value. In a typical human, normal fasting serum beta-hydroxybutyrate (BHB) range from 0.01 mM to 0.2 mM. Therefore, ketosis or ketogenic state can be considered to be serum BHB levels greater than 0.2, mM, 0.3 mM, or 0.4 mM, with increasing preference, lasting for one hour or more.

Ketosis or the ketogenic state can be achieved by several means including diet, physical training regimen, and/or the administration of agents that increase fatty acid oxidation.

When the ketogenic treatment is dietary, it typically comprises the reduction of dietary carbohydrates and the increase of dietary lipids. The classic ketogenic diet uses low carbohydrate and low protein, such that 90% of the calories come from fat. The rational for this is that ingestion of protein elevates serum amino acid levels which will stimulate the release of insulin and suppress the ketogenic state. However, the effect of protein is minor and in practice, protein does not have to be limited. Ketosis can be achieved when consuming a high protein diet, provide carbohydrate content is low. Thus, ketogenic treatment by diet means increased lipid intake, with carbohydrate restriction alone, or also in combination with protein restriction. The reduction of carbohydrates reduces serum insulin signaling, which in turn alters protein metabolism.

As discussed herein, the reduced insulin signaling produced by the ketogenic treatment of the subject invention results in reduced protein synthesis and increased protein degradation. Active insulin signaling promotes whole body protein synthesis and inhibits protein degradation. For example, decreasing insulin levels in rats reduces protein synthesis 40% in muscle cells (for review see Liu and Barrett, Am J Physiol Endocrinol Metab, 2002, 283:E1105-12). The reduction in protein synthesis occurs through regulation of the initiation of protein translation. This regulation occurs at the 5' end of the messenger RNA and affects the assembly of the ribosomal complex at the AUG start site. This regulation occurs largely through modification of two key proteins, ribosomal protein S6 kinase (S6K1) and eukaryotic initiation factor 4E binding protein (EF-BP1). S6K1 is a protein kinase that phosphorylates ribosomal protein S6. Phosphorylated S6 increases the translation of a set of messenger RNAs that encode translational machinery, hence activation of S6 results in increased ribosome number and a general increase in protein synthesis. EF-PB1 acts as an inhibitor of protein synthesis by binding to eIF4E and thereby preventing assembly of translational machinery.

The kinase cascade initiated by insulin signaling leads to modification of the phosphostate of both S6K1 and EF-4BP. Insulin signaling stimulates phosphorylation of S6K1, increasing its activity and elevates levels of phosphorylated S6 . Insulin signaling also leads to the phosphorylation of EF-4BP, disrupting the interaction with eIF4E and thereby allowing eIF4E to participate in assembly of translational complex (Shah, et al., Am J Physiol Endocrinol Metab, 2000, 279:E715-29). Together, these two mechanisms result in the potentiation of protein synthesis by insulin signaling.

The mechanism whereby insulin signaling inhibits protein degradation is poorly understood. Yet, it is clear that withdrawal of insulin promotes protein degradation. The increase in proteolysis upon insulin withdrawal may involve up regulation of ubiquitin and proteosome components or possibly inactivation of the TOR kinase (for overview see Kimball, et al., J Appl Physiol, 2002, 93:1168-80; Liu and Barrett, Am JPhysiol Endocrinol Metab, 2002, 283:E1105-12).

Decreasing insulin signaling also increases cellular lipid metabolism, which improves lipid homeostasis within neurons and improves function of lipid sensitive proteins such as APP. For example, despite the importance of fatty acids in cerebral neurons, little *de novo* fatty acid synthesis occurs in the adult brain. Most fatty acids are imported as phospholipids or unesterified free fatty acids from the plasma through the use of fatty acid transport proteins. One important class of fatty acids required by the CNS are essential fatty acids (EFAs). EFAs, such as docosahexanoic acid (DHA) are found extensively in phospholipids of neuronal membranes. Inhibition of lipid metabolism by insulin signaling will decrease serum EFA levels and lead to substitution of non-ideal fatty acids into lipid membranes. This alteration of membrane composition will disturb the activity and functioning of membrane proteins. Administration of a ketogenic treatment will lower insulin signaling and increase serum EFA levels thereby promoting proper neuronal membrane composition.

One important protein that is sensitive to disturbances in lipid homeostasis is APP. Excess cholesterol and altered phospholipid composition of membranes leads to increased aberrant cleavage of APP and failure of APP function. Hence, a low carbohydrate diet will lead to elevated serum EFA levels and improved APP function within neurons. In a preferred embodiment of the dietary ketogenic treatment, the carbohydrate levels are restricted to an amount, in increasing preference, of less than 20%, 15%, 10%, 5% or 2% of total calories.

In an alternative embodiment, the ketogenic treatment may involve administration of an agent that increases fatty acid oxidation alone or in combination with other ketogenic treatments. Examples of agents that increase utilization of fatty acids may be selected from a group consisting of, but not limited to, L-carnitine, caffeine, ephedra alkaloids, non-steroidal anti-inflammatory agents (NSAIDs), statin drugs (such as Lipitor® and Zocor®) and fibrates.

Carnitine is used to transport fatty acid moieties into the mitochondria for oxidation. Thus, in the present invention, a ketogenic treatment can comprise administration of carnitine (in doses required to increase the utilization of fatty acids) alone or in combination with another ketogenic treatment. The dosage of L-carnitine will vary according to the condition of the host, method of delivery, and other factors known to those skilled in the art, and will be of sufficient quantity to raise blood ketone levels to induce a ketogenic state. Derivatives of L-carnitine which may be used in the present invention include but are not limited to decanoylcarnitine, hexanoylcarnitine, caproylcarnitine, lauroylcarnitine, octanoylcarnitine, stearoylcarnitine, myristoylcarnitine, acetyl-L-carnitine, O-Acetyl-L-carnitine, and palmitoyl-L-carnitine.

Caffeine and ephedra alkaloids are commonly used in over the counter diet supplements. Ephedra alkaloids are commonly derived from plant sources such as ma-huang (Ephedra Sinica). The combination of caffeine and ephedra stimulate the use of fat. Ephedra alkaloids are similar in structure to adrenaline and activate beta-adenergic receptors on cell surfaces. These adenergic receptors signal through cyclic AMP (cAMP) to increase the use of fatty acids. cAMP is normally degraded by phosphodiesterase activity. One of the functions of caffeine is to inhibit phosphodiesterase activity and thereby increase cAMP mediated signaling. Therefore caffeine potentiates the activity of the ephedra alkaloids. Accordingly, the present invention comprises the use of ephedra alkaloids alone or in combination with another ketogenic treatment to reduce the cellular accumulation of toxic proteins. Additionally, the invention can comprise the use of ephedra alkaloids in combination with caffeine for the treatment or prevention of cellular protein aggregates and conditions associated therewith. Furthermore, the invention can comprise the use of ephedra alkaloids and caffeine in combination with another ketogenic regime to provide a treatment or prevention of cellular protein aggregates and conditions associated therewith.

NSAIDs function, in part, as PPAR-gamma agonists. Increasing PPAR-gamma activity increases the expression of genes associated with fatty acid metabolism such as FATP. Accordingly, a combination of PPAR-gamma agonists and another ketogenic treatment can prove beneficial to individuals with protein aggregation diseases. In a preferred embodiment, the PPAR-gamma agonist is an NSAID. Examples of NSAIDs include: aspirin, ibuprofen (Advil, Nuprin, and others), ketoprofen (Orudis KT, Actron), and naproxen (Aleve).

Statins are a class of drugs with pleiotropic effects, the best characterized being inhibition of the enzyme 3-hydroxy-3-methylglutaryl CoA reductase, a key rate step in cholesterol synthesis. Statins also have other physiologic affects such as vasodilatory, antithrombotic, antioxidant, anti-proliferative, anti-inflammatory and plaque stabilizing properties. Additionally, statins cause a reduction in circulating triglyceride rich lipoproteins by increasing the levels of lipoprotein lipase while also decreasing apolipoprotein C-III (an inhibitor of lipoprotein lipase) (Schoonjans, et al., FEBS Lett, 1999, 452:160-4). Accordingly, administration of statins results in increased fatty acid usage, which can act synergistically with other ketogenic treatment. Thus, one embodiment of this invention would be combination therapy consisting of statins and other ketogenic treatment.

Fibrates, such as Bezafibrate, ciprofibrate, fenofibrate and Gemfibrozil, are a class of lipid lowering drugs. They act as PPAR-alpha agonists and similar to statins they increase lipoprotein lipase, apoAI and apoAII transcription and reduce levels of apoCIII. As such they have a major impact on levels of triglyceride rich lipoproteins in the plasma, presumably by increasing the use of fatty acids by peripheral tissues. Accordingly, the present invention includes the use of fibrates alone or in combination with other ketogenic treatment to reduce cellular protein aggregation which can be beneficial to patients having diseases related thereto.

In a further embodiment, the ketogenic treatment is induced by a physical training regimen. Physical activity can also lower insulin signaling and increase the utilization of fats. Physical activity is fueled by a mix of carbohydrates (glucose) and fats. The ratio of carbohydrate to fat depends on many factors and changes over time. Typically, glucose is used preferentially in the first phase of exercise, while fat utilization typically increases after 20-30 minutes of sustained activity. Depending on the intensity of the work performed glucose uptake by muscles rises 7 to 20 times over the basal level. Sustained activity, defined as greater than 50% of max heart rate (220-age = max heart rate) or VO2Max (maximal oxygen consumption) for at least 20 minutes, will cause a decrease in insulin levels and a shift toward fatty acid metabolism. In addition, intense exercise provokes the release of insulin-counter regulatory hormones such as glucagons and catecholamines, which ultimately cause a reduction in the insulin action (Sato, et al., Exp Biol Med (Maywood), 2003, 228:1208-12). Hence it is the novel insight of the inventor that sustained physical activity for at least 20 or 30 minutes will induce a ketogenic state and prevent and treat protein aggregation diseases.

The ketogenic treatment can also involve a combination of at least two treatments selected from the group consisting of dietary treatment, administration of fatty acid oxidation agents, and physical training regimen. Most preferred is the combination of the dietary treatment and the physical training regimen.

The subject method is used to reduce the amount of protein aggregates in neurons. The aggregates can comprise amyloid β peptide, polyglutamine containing huntintin protein, polyglutamine containing androgen receptor, polyglutamine containing atrophin-1, polyglutamine containing ataxins, α-synclein, prion protein, tau and/or superoxide dismutase 1 (SOD1).

One or more of the methods according to the present invention can be administered along with other compounds known to be useful for the treatment of protein aggregation diseases. Such compounds include but are not limited to neuroprotective compounds and compounds which inhibit aggregate formation or inhibit protein aggregation. Examples of such compounds include but are not limited to minocycline, ethyl eicosapeninoate, riluzole, Congo red, cysteamine and cystamine.

The predetermined period of treatment varies according to the severity of the patient's condition, patient weight, and other factors known to persons skilled in the art. In one embodiment, the ketogenic treatment is administered or undertaken for 6 to 12 months. Alternately, the ketogenic treatment can be administered or undertaken for 1 to 6 months. Other treatment periods are for one day to one month. Treatment periods can also be periodic, e.g., alternating one week on the ketogenic treatment and one week off.

The subject invention also comprises an assay for measuring the effectiveness of ketogenic treatment for the reduction or prevention of protein aggregation in cells including neurons. This assay comprises treating cells *in vivo* or *in vitro* with a ketogenic treatment and determining whether such treatment prevents or reduces protein aggregation in the cells relative to untreated cells. The reduction in protein aggregation is, with increasing preference, at least 5%, 10%, 15%, 20%, 25%, etc. by weight in increasing increments of 5%. In one embodiment, the *in vivo* treatment involves ketogenic treatment of a mammal (preferably non-human) that expresses neuronal aggregation of a protein associated with a protein aggregation disease, such as Alzheimer's disease, Parkinson's disease, polyglutamine diseases, prion diseases, taupathologies, and familial amyotrophic lateral sclerosis. Example 1 exemplifies the APP/V717I transgenic mouse model for Alzheimer's disease, which is useful in determining the effectiveness of a ketogenic treatment in reduction or prevention of Aβ protein. Likewise, Example 3 exemplifies a transgenic mouse carrying a mutant polyQ transgene, such as exon 1 of the Huntingin protein, which is useful in determining the effectiveness of a ketogenic treatment in preventing or reducing protein aggregation and deterioration of neuronal function. In another embodiment, the *in vitro* treatment involves ketogenic treatment of cultured cells which exhibit protein aggregation, to determine whether such treatment reduces or prevents protein aggregation as compared to cells that do not receive the ketogenic treatment. Example 2 exemplifies an *in vitro* cellular assay for effectiveness of ketogenic treatment.

All references cited herein are incorporated in their entirety by reference.

### BRIEF DESCRIPTION OF THE FIGURES

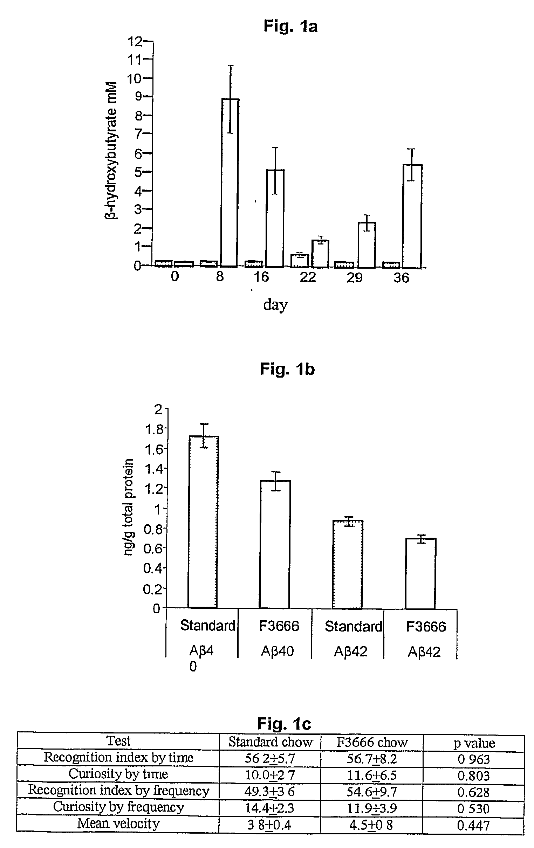

Fig. 1a illustrates the effect of the ketogenic diet in a transgenic mouse model of Alzheimer's disease as described in Example 1. Standard diet is shown as grey bars, ketogenic diet is shown as white bars, and error bars represent standard error of the mean. Serum β-hydroxybutyrate levels are provided in mM. "Day" represents time, in days, from change of diet. Serum β-hydroxybutyrate levels were significantly elevated at start of food change and animals on ketogenic chow lost weight. To mitigate weight loss and improve feeding a small amount of standard chow was mixed with F3666 during the second week and then removed and ketone levels increased.
Fig. 1b shows Aβ levels in ng/g of total protein of the mouse model as described in Example 1. Again standard diet is shown as grey bars, ketogenic diet is shown as white bars, and error bars represent standard error of the mean.
Fig. 1c illustrates results of behavioral testing as described in Example 1.

### DETAILED DESCRIPTION OF THE INVENTION

It is the novel insight of the inventor that the effects of reduced insulin signaling on both lipid and protein metabolism can be useful in the treatment and prevention of protein accumulation in cells and in the treatment of diseases related thereto.

As discussed herein, ketogenic diets can be useful in reducing neuron protein aggregation because ketogenic diets alter both lipid and protein metabolism. For example, low insulin signaling promotes protein degradation while inhibiting protein synthesis. It is the novel insight of the inventor that reduced insulin signaling can lead to the clearing of degradation sensitive proteins, such as amyloidic peptides and mutant Huntington proteins. In addition, lower insulin signaling favors lipid metabolism over glucose use, and can lead to improved lipid homeostasis within neurons and improved function of lipid sensitive proteins such as APP. Together these mechanisms will offer treatment for protein aggregation diseases.

Contrary to the current belief that high fat diets increase risk for AD, the inventor has proposed that high carbohydrate (HC) diets are the primary cause of AD (Henderson, Med Hypotheses, 2004, 62:689-700). This analysis is based on many factors including the known risk factors for sporadic AD, the most common form of the disease (>95% of cases). Sporadic AD is not linked with mutations in APP or presenilin genes, instead it is associated with genetic risk factors. The best characterized risk factor for sporadic AD is possession of one or more of the ε4 alleles of the apolipoprotein E gene. Demographic analysis of the ε4 allele has revealed that ε4 is rare in populations with long historical exposure to agriculture, suggesting consumption of HC diet may have selected against ε4 carriers (Corbo and Scacchi, Ann Hum Genet, 1999, 63 (Pt 4):301-10). Furthermore, the ApoE4 protein inhibits lipid metabolism in a manner similar to consumption of a high carbohydrate diet. Therefore, it has been proposed that the inhibition of lipid metabolism by both HC diets and ApoE4 is central to the development of AD (Henderson, Med Hypotheses, 2004, 62:689-700).

Dietary intervention represents a relatively safe and readily available method to combat AD. Yet, the key dietary links have previously remained unclear. Much of the earlier work has focused on the role of high fat / cholesterol diets and their contribution to AD. Previous studies had suggested that high fat / high cholesterol diets increased both the production and deposition of Aβ in mouse models of AD, leading to the suggestion that diets rich in lipids were a factor in AD (George, et al., Neurobiol Dis, 2004, 16:124-32;Refolo, et al., Neurobiol Dis, 2000, 7:321-31;Shie, et al., Neuroreport, 2002, 13:455-9). However, these diets were not low carbohydrate diets. In the high cholesterol diets cholesterol was added to the diet without reduction in other components (Refolo (2000), supra; George (2004), supra). Other high fat diets were fat rich diets that did not significantly reduce carbohydrate levels and hence were not low carbohydrate diets. Ho *et al* used a diet of 60% fat, 20% carbohydrate, 20% protein. This diet was sufficiently high in carbohydrate to cause the large increases in body weight (Ho (2004), supra). In the present invention carbohydrate intake was very low (<1%) and resulted in weight loss. Hence, the present invention describes that novel finding that it is not fats in the diet that cause increased Aβ levels, but perhaps levels of carbohydrate or total calories.

Other studies have examined the role of carbohydrate content in the diet and Aβ deposition. Wang *et al.* examined transgenic AD mice under *ad libitum* feeding and a caloric restriction regime in which carbohydrate content and total calories were reduced 30%. This treatment began when the animals were 3 months of age and the animals examined at 12 months of age. The low carbohydrate low calorie group had significantly lower plaque formation (Wang, et al., Faseb J, 2005, 19:659-661). The present invention is distinct from Wang *et al.* in many ways: 1. Wang *et al.* used a caloric restriction regime, where the total calories were limited in the experimental group. In the present invention animals had free access to food at all time and calories were not limited. 2. Wang et al did not use a ketogenic treatment, instead carbohydrate levels were still a significant portion of the diet and not low enough to promote ketosis. 3. Wang *et al.* administered caloric restriction for 9 months. Typically long exposure to caloric restriction is necessary to see effects. In the present invention treatment was administered for only 38 days. In summary, it is the novel and surprising insight of inventor that a brief (38 day) very low (<1%) carbohydrate, high fat treatment is sufficient to reduce Aβ levels by 25%. Such results represent an unexpected efficiency in reduction of cellular protein aggregation.

In US patent application US 2004/0058873 A1, Esmond et al.*,* teach that low carbohydrate diets can be used to treat Alzheimer's disease. Paragraph [0031] states:
A second method of the invention is directed to the treatment or prevention or Alzheimer's disease by the restriction of metabolizable carbohydrate in the diet. According to the invention, the amount of metabolizable carbohydrate is considered restricted if no more than about 55 grams are ingested per day. Preferably, no more than about 30 grams of metabolizable carbohydrate are ingested. Most preferably, no more than about 10 grams of metabolizable carbohydrate are ingested.

The present invention differs from the teachings of 0058873 in the following ways.
1. The present invention relates to the reduction of levels of aggregated proteins, while US 2004/0058873 relates to treatments for Alzheimer's disease by unclear mechanisms. The toxicity of Aβ peptides remains an unanswered question in AD and it is not clear from Esmond *et al.* that their treatment would reduce Aβ levels. Furthermore, it is not clear that reduction of Aβ levels would necessarily treat AD. In contrast, the present invention is directed to reduction of protein aggregation in cells.
2. The present invention represents clear advantages over US 2004/0058873, which merely states that carbohydrates are to be lowered, but provides no means to measure the efficacy of the treatment. The present invention teaches that elevated serum ketone body levels are a marker for a ketogenic state that can be used to monitor compliance with the prescribed ketogenic treatment.
3. Furthermore, the present invention represents an improvement over US 2004/0058873 in that the present invention provides means to measure effectiveness of treatment. The present invention teaches that ketogenic treatments can reduce Aβ levels and *in vivo* or *in vitro* measurements of Aβ levels can be an effective means to measure ketogenic treatment efficacy. Methods to detect levels of Aβ in sera are well known to those skilled in the art. Measuring Aβ levels *in vivo* within the CNS is also known to those skilled in the art (Nordberg, Lancet Neural, 2004, 3:519-27). Detection of other aggregrated proteins is well known in the field. For example detection of polyglutamine inclusions is well documented (Yamamoto, et al., Cell, 2000, 101:57-66).
4. US 2004/0058873 also relates to treatment of AD with insulin sensitizing agents. Paragraph [0033] states:
   The present invention also relates to a method improving mentation of a patient with Alzheimer's disease, comprising administration to said patient an effective amount of an agent which increases insulin sensitivity of the patient.
In the present invention, administration of a high fat diet is well known to those skilled in the art to induce insulin resistance and NOT insulin sensitivity. Insulin resistance also commonly occurs during starvation, a condition mimicked by ketogenic diet. Under both of these conditions (high fat diet and starvation), tissues such as muscle and liver become resistant to the action of insulin and hence decrease their uptake of glucose. This is a mechanism to preserve glucose for central nervous system function. It also has many other outcomes. Inducing insulin resistance inhibits insulin signaling which inhibits protein synthesis, while increasing protein degradation (as previously discussed herein). It is the novel insight of the inventor that these alterations in protein metabolism are advantageous to decrease levels of toxic proteins. Hence, decreased insulin sensitivity, via a ketogenic treatment, will be the preferred mechanism to treat protein aggregation diseases. This is the opposite of what is taught in US 2004/00558873.

In one embodiment of the subject invention, the mammal is administered a low carbohydrate, low protein ketogenic diet that maintains the mammal in a state of ketosis. Ketosis is defined as serum ketone body levels elevated above normal fasting values. Such values will vary depending on both the species of mammal and individual within a species. In humans, normal fasting serum beta-hydroxybutyrate (BHB) range from 0.01 mM to 0.2 mM and can rise as high as 14 mM during prolonged starvation. Therefore BHB levels above 0.3 mM for more than one hour can be considered to be ketosis or a ketogenic state in humans. In *ad libitum* fed mice, typical BHB levels range from 0.01 to 0.4 mM (see Example 1, below). Induced ketone levels in mice range from 0.4 to as much as 10 mM. Therefore, in mice, ketosis can be considered to represent serum BHB levels above 0.4 mM for longer than one hour.

Ketone bodies include acetoacetate, β-hydroxybutyrate and acetone. Methods to measure serum and tissue ketone levels are well known to those skilled in the art.

For a human, the subject invention would require that carbohydrate levels be restricted to less than twenty percent of total calories. For example, for an individual on a 2000 a day calorie diet, less than 400 calories a day could be derived from carbohydrate (1 gram of carbohydrate = 4 calories); therefore, less 100 grams a day of carbohydrate would be consumed. More ideally, less than 50 grams a day, and even more preferred is less than 10 grams a day. Variation in diet composition will be necessary to maintain said mammal in ketosis. For example, progressively more carbohydrate may have to be restricted over time to maintain ketosis.

In another embodiment, agents that induce a ketogenic state are administered to a mammal in need thereof. A ketogenic state is defined as serum ketone body levels elevated above normal fasting values, but which are induced by non-dietary means. A ketogenic state would differ from ketosis in that it may be achieved by methods other than dietary intervention, such as administration of a compound or a physical training regime. For example, administration of agents that increase fatty acid oxidation may induce a ketogenic state even in the presence of abundant carbohydrates. Such a state may prove beneficial in the treatment of protein aggregation diseases.

In one preferred embodiment, a carbohydrate restricted diet is combined with an exercise program to decrease insulin signaling, improve fatty acid oxidation, and increase blood ketone levels.

In another embodiment, a brief ketogenic treatment is administered to a mammal in need thereof. This treatment could be either induction of ketosis by dietary intervention or induction of ketogenic state as described above. Such a treatment would be of limited duration, ranging from 1 day to 1 year, after which time said mammal would return to feeding *ad libitum* or discontinue treatments. Macronutrient components of the ketogenic diet would be as described above, with precise profile depending on species and individual.

In another preferred embodiment the success of a treatment for dementia is determined based on blood ketone levels. Compounds or interventions to treat or prevent dementia will be assayed by their ability to induce elevated serum ketone body levels above baseline values.

Example 1 demonstrates that a ketogenic diet is successful in reducing the levels of Aβ peptide in a transgenic mouse model of Alzheimer's disease. After 40 days of treatment, Aβ levels were decreased 25% without adverse consequences on cognitive performance. These results are surprising in that they run contrary to the current view linking high fat diets with Alzheimer's disease.

The following examples are intended to illustrate but not limit the present invention.

### EXAMPLES

### Example 1

Here we tested the effects of an extremely low carbohydrate / high fat ketogenic diet on a transgenic mouse model of AD, APP/V717I (Moechars, et al., JBiol Chem, 1999, 274:6483-92). Sixteen APP/V717I mice were raised on standard chow for three months, then half were switched to a low carbohydrate / high fat chow, Bio Serv F3666 (6:1 ratio of fats:carbohydrate, protein (Bio-Serv Inc.)) and the remaining 8 mice remained on standard diet (RM-Klaver).

| **Contents** | **RM-Klaver** | **F3666** |
|---|---|---|
| Carbohydrate | 35% | 0.76% |
| Protein | 21% | 8% |
| Fat | 4.5% | 79% |
| Water/ash/fiber | 39.5% | 12.24% |

To measure the effectiveness of the chow, blood samples were taken weekly and examined for serum β-hydroxybutyrate (BHB) levels (Stanbio β-hydroxybutyrate kit, StanBio Inc.). Over the course of the experiment animals in the F3666 group had greatly elevated serum BHB levels compared to standard chow (Standard 0.323 ± 0.406 mM vs. F3666 3.976 ± 0.420 mM, p<0.0001).

After four weeks on the diet animals were tested for behavioral deficits using object recognition tests (ORT). Contrary to previous results (Zhao, et al., Pediatr Res, 2004, 55:498-506), there was no worsening of cognitive performance in the group fed high fat chow (See Figure 1c).

Cognitive performance was measured using the novel object recognition test. This test was performed after 38 days of treatment and three days before sacrifice. The protocol used followed the method as described by Dewachter *et al.* (Dewachter, et al., J Neurosci, 2002, 22:3445-53). Mice were familiarized for one hour to a Plexiglas open-field box (52 x 52 x 40 cm) with black vertical walls and a translucent floor, dimly illuminated by a lamp placed underneath the box. The next day the animals were placed in the same box and submitted to a 10 minutes acquisition trial. During this trial mice were placed individually in the open field in the presence of 2x object A (orange barrel or green cube, similar sized of ± 4 cm), and the duration (time_{AA}) and the frequency (Freq_{AA}) exploring object A (when the animals snout was directed towards the object at a distance of < 1 cm and the mice were actively sniffing in the direction of the object) was recorded by a computerized system (Ethovision, Noldus information Technology, Wageningen, the Netherlands). During a 10 minutes retention trial (second trial) performed 3 hours later, a novel object (object B, green cube or orange tun) was placed together with the familiar object (object A) into the open field. (Freq _{A} and Freq _{B} and Time_{A} and Time_{B}, respectively).

The recognition index (RI), defined as the ratio of the duration in which the novel object was explored over the duration in which both objects were explored [Time _{B} / (Time _{A} + Time _{B}) x 100], was used to measure non-spatial memory. The duration and frequency object A was explored during the acquisition trial (Time_{AA} and Freq_{AA}) was used to measure curiosity.

Mice that do not distinguish between an old object and a new one, have a recognition index of 50. Mice that recognize the old object, will preferably explore the novel object and hence the recognition index becomes > 50. Mice that exclusively explore the novel object have a recognition index of 100. After 38 days of treatment there was no difference in any ORT scores (figure 1c).

At the conclusion of the experiment both serum and brain BHB measures were obtained. Consistent with earlier results, serum BHB levels were higher in F3666 fed animals (Standard 0.12 ± 0.618 mM vs. F3666 3.26 ± 0.667 mM, p=0.00541).

Contrary to early reports of high fat diets increasing Aβ levels in the brain, levels of both soluble Aβ 40 (Standard 1.725 ± 0.104 vs F3666 1.279 ± 0.112 ng/g protein, p value = 0.0140) and Aβ 42 (Standard 0.879 ± 0.042 vs F3666 0.706 ± 0.046 ng/g protein, p value = 0.016) were found to be significantly lower in the brains of the F3666 fed group, while overall protein levels as measure as mg/ml of brain homogenate did not differ (Standard 0.562 ± 0.035 vs. F3666 0.509 ± 0.017, p= 0.213). There was no difference in the mean ratio of Aβ 42/40 between groups (Standard 0.517± 0.024 vs. F3666 0.556±0.026, p= 0.2872) suggesting a general lowering of Aβ species but not specific to either form.

### Example 2

A cell-based assay is used to show that ketogenic conditions, low glucose and low growth factor levels, can decrease toxic protein levels. Differentiated inducible PC 12 cells expressing a polyQ green fluorescent protein (polyQ::GFP) transgene are tested for visible polyQ::GFP protein inclusion formation under normal growth conditions and ketogenic conditions. Cells are plated on 10-cm plates using standard tissue culture medium including abundant glucose and growth factors. Examples of such media include Dulbecco's Modified Eagle Medium (D-MEM) (1X) liquid (high glucose); such media contains 4500 g/L (25 mM) glucose and are supplemented with fetal calf serum rich in growth factors (> 1 nM insulin/IGF-1). Cells are allowed to express the polyQ::GFP protein and to form inclusions. After inclusions have formed, half the plates are maintained in normal media, while half are exposed to ketogenic media (low glucose and low growth factor). Examples of ketogenic media include Minimum Essential Medium (MEM) liquid (Life technologies Inc.); such media contains 1000 mg/L (5.56 mM) glucose and does not contain growth factors. Thus media containing less than 5.56 mM glucose can be considered low glucose and ketogenic. Growth factors, such as insulin, are known to inhibit the activity of ketogenic enzymes. For example, insulin levels greater than 1 nM were found to inhibit the expression of key enzymes in ketogenesis (Nadal, et al., Biochem J, 2002, 366:289-97). Thus ketogenic media can be considered media in which growth factors, such as insulin are below 1 nM. The cells are grown overnight in the differential media and aggregation is calculated by using fluorescent microscopy. It is expected that ketogenic media will be found to inhibit aggregation and reduce levels of polyQ::GFP.

### Example 3

Mice carrying a mutant polyQ containing transgene are used to show that a brief ketogenic diet treatment reduces polyglutamine *pathogenesis in vivo.* Mice that carry a transgene expressing exon 1 of the Huntingin protein with a polyQ coding region develop progressive motor dysfunction, neuronal inclusions, and neuropathology typical of HD. Such transgenic animals are raised on normal, high carbohydrate, rodent chow until the age they typically begin to show signs of motor dysfunction. At this time, half of the mice are switched to a ketogenic chow (as described in Example 1) while half are left on normal chow. Mice in each group are maintained for 30 days on their respective diets. At the end of the treatment, mice in each group are tested for motor function, using a rotating rod. After completion of motor testing, the brains of the animals are examined for the presence and extent of neuronal inclusions. Mice fed ketogenic chow are expected to perform for longer times on the motor rod, reflecting significant rescue of motor function, and show decreased amount and extent of polyQ containing aggregates.

The following numbered paragraphs (paras.) contain statements of broad combinations of the inventive technical features herein disclosed:-
1. A method of reducing protein aggregation in the brain of a mammal, said method comprising administering a ketogenic treatment so to produce ketosis or a ketogenic state in the mammal for a predetermined period, whereby the protein aggregation is reduced.
2. The method of paragraph 1, wherein the ketosis or ketogenic state comprises maintenance of a serum ketone body level elevated above normal fasting value.
3. The method of paragraph 2, wherein serum ketone level is above 0.2 mM, 0.3 mM, 0.4 mM or higher for one hour or more.
4. The method of paragraph 1, wherein the ketogenic treatment is dietary and comprises:
   a) reducing dietary carbohydrates whereby serum insulin level and protein synthesis is reduced, while protein degradation is increased; and
   b) increasing dietary lipids whereby cellular lipid metabolism is increased.
5. The method of paragraph 4, wherein the carbohydrate levels are restricted to an amount less than 20%, 10% or 2% of total calories and serum ketone levels are elevated above normal fasting baseline levels.
6. The method of paragraph 1, wherein the ketogenic treatment is induced by an agent that increases fatty acid oxidation.
7. The method of paragraph 6, wherein the agent that increases fatty acid oxidation is selected from the group consisting of L-carnitine and derivatives thereof, caffeine, ephedra alkaloids, non-steroidal anti-inflammatory agents (NSAIDs), statin drugs, and fibrates.
8. The method of paragraph 1, wherein the ketogenic treatment is induced by a physical training regimen.
9. The method of paragraph 8, wherein the physical training regimen comprises activity above 50% of either VO2max or maximal heart rate for a period of greater than 20 minutes.
10. The method of paragraph 1, wherein the ketogenic treatment is induced by a combination of at least two treatments selected from the group consisting of dietary treatment, fatty acid oxidation agent treatment, and physical training regimen.
11. The method of paragraph 10, wherein the dietary treatment is combined with the physical training regimen.
12. The method of paragraph 1, wherein the protein aggregates are selected from the group consisting of amyloid β peptide, polyglutamine containing huntintin protein, polyglutamine containing androgen receptor, polyglutamine containing atrophin-1, polyglutamine containing ataxins, α-synclein, prion protein, tau and superoxide dismutase 1 (SOD1).
13. The method of paragraph 1, further comprising administering a second compound which inhibits aggregate formation or is neuroprotective.
14. The method of paragraph 13, wherein the second compound is selected from the group consisting of Congo red, cystamine, cysteamine, minocycline, ethyl eicosapentaenoate, and riluzole.
15. The method of paragraph 1, wherein the ketogenic treatment is between 1 day and 1 year.
16. An assay for measuring the effectiveness of ketogenic treatment for the reduction or prevention of protein aggregation in cells, comprising
   a) treating said cells *in vivo* or *in vitro* with a ketogenic treatment; and
   b) determining whether such treatment prevents or reduces protein aggregation in the cells.
17. The method of paragraph 16, wherein the reduction in protein aggregation is at least 25% by weight.
18. The assay of paragraph 16, wherein the *in vivo* treatment involves ketogenic treatment of a non-human mammal that exhibits neuronal aggregation of a protein associated with a protein aggregation disease under non-ketogenic conditions.
19. The assay of paragraph 16, wherein the non-human mammal is a transgenic mouse that exhibits neuronal protein aggregation under non-ketogenic conditions.
20. The assay of paragraph 16, wherein the *in vitro* treatment comprises ketogenic treatment of cultured cells which exhibit protein aggregation under non-ketogenic conditions.

## Claims

1. A low carbohydrate/high fat chow for use in a method of reducing protein aggregation in the brain of a mammal, said method comprising inducing ketosis or a ketogenic state in the mammal for a predetermined period, whereby the protein aggregation is reduced.

2. The low carbohydrate/high fat chow for use according to claim 1, wherein the ketosis or ketogenic state comprises maintenance of a serum ketone body level elevated above normal fasting value.

3. The low carbohydrate/high fat chow for use according to claim 2, wherein serum ketone level is above 0.2 mM, 0.3 mM, 0.4 mM or higher for one hour or more.

4. The low carbohydrate/high fat chow according to claim 3, wherein the carbohydrate levels are restricted to an amount less than 20%, 10% or 2% of total calories and serum ketone levels are elevated above normal fasting baseline levels.

5. The low carbohydrate/high fat chow for use according to claim 1, wherein the chow comprises a 6:1 ratio of fats:carbohydrate.

6. The low carbohydrate/high fat chow according to claim 1, wherein the method further comprises administering an agent that increases fatty acid oxidation.

7. The low carbohydrate/high fat chow according to claim 6, wherein the agent that increases fatty acid oxidation is selected from the group consisting of L-carnitine and derivatives thereof, caffeine, ephedra alkaloids, non-steroidal anti-inflammatory agents (NSAIDs), statin drugs, and fibrates.

8. The low carbohydrate/high fat chow according to claim 1, wherein the protein aggregates are selected from the group consisting of amyloid β peptide, polyglutamine containing huntintin protein, polyglutamine containing androgen receptor, polyglutamine containing atrophin-1, polyglutamine containing ataxins, α-synclein, prion protein, tau and superoxide dismutase 1 (SOD1).

9. The low carbohydrate/high fat chow according to claim 1, wherein the method further comprises administering a second compound which inhibits aggregate formation or is neuroprotective.

10. The low carbohydrate/high fat chow according to claim 9, wherein the second compound is selected from the group consisting of Congo red, cystamine, cysteamine, minocycline, ethyl eicosapentaenoate, and riluzole.

11. The low carbohydrate/high fat chow according to claim 1, wherein the treatment is between 1 day and 1 year.
